# EUROPEAN PATENT SPECIFICATION

(11) **EP 0 399 518 B1**
(45) Date of publication and mention of the grant of the patent: **29.12.1993**
(21) Application number: 90109846.7
(22) Date of filing: 23.05.1990
(51) Int. Cl.: C08L 21/00, A61L 15/44, A61L 15/60, A61K 47/32

(54) **Water swellable composite rubbers and their preparation and use**
In Wasser quellfähige Kautschukzusammensetzungen, deren Herstellung und Verwendung
Compositions de caoutchouc gonflables dans l'eau, leur preparation et leur utilisation

(30) Priority: 24.05.1989 CS 3127/89
(43) Date of publication of application: 28.11.1990
(73) Proprietor: Ceskoslovenska akademie ved, Praha 1 (CS)
(72) Inventor: Vondrácek, Petr, Praha (CS); Lopour, Petr, Praha (CS); Sulc, Jiri, Praha (CS)
(74) Representative: Beetz & Partner Patentanwälte

(56) References cited:
- EP-A- 0 118 998
- EP-A- 0 179 937
- DE-A- 2 162 395
- FR-A- 2 582 009

## Description

The invention pertains to composite rubbers swellable with water and aqueous solutions, and to methods for their production and their use.

Rubbers used for technical purposes until now are all hydrophobic polymeric materials which do not swell in water, and the surface of which is not wettable with water unless they undergo a special finishing. At the same time, water swellable elastic polymeric materials would be suitable for many technical purposes, for example, for manufacturing rubber parts with which sealing could be attained after swelling with water, or for manufacturing materials which could be used as sensors and functional parts of controlling systems, or where the swelling pressure could be used for a simple indication of the water content in various media and materials, and the like.

Silicone elastomers known from CS-A-251 890 (corresponding to FR-A-2 582 009) are able to swell in water and aqueous solutions and have a surface wettable with water. These polymer composites contain 10 to 150 mass parts of a powdery hydrogel filler pro 100 mass parts of a silicone polymer forming the continuous matrix of the composite. In the dry state, these composite materials have the apearance and properties of normal silicone rubber, but they are able to swell in water and aqueous solutions and contain up to 90 mass-% of water if swollen to equilibrium. It has been proved that the water swollen materials of this type are neither irritating nor toxic and are able to dissolve low-molecular and medium-molecular mass water-soluble compounds, including drugs, which makes possible their application in medicine as implants or for controlled drug release and intradermal drug forms. An important property of these materials is their shape memory, which permits to change the shape of objects made of them, after heating above a certain temperature, or after swelling in water or aqueous solutions, in a defined way.

The invention is based on the finding that powdery hydrogel fillers may be advantageously used also for the preparation of water-swellable rubbers based on various other synthetic rubbers.

It is the object of the invention to provide rubbers swellable with water or aqueous solutions, which consist of a vulcanised rubber matrix based on rubbers vulcanizable at temperatures up to 150 °C, and preferably of 15 to 150 °C, and one or more hydrogel fillers dispersed therein, methods for their preparation, and their use.

DE-A2-2 162 395 discloses a method for the production of thin articles from plastics or rubbers additionally containing fillers on the basis of synthetic crosslinked water-soluble macromolecular compounds. These materials, however, are not water-swellable, because they contain up to 20 % of a crosslinking agent.

In EP-A-0 118 998 there is disclosed a water-swelling rubber composition comprising a chloroprene rubber, a highly water absorptive resin, a rubbery polymer unvulcanizable with metal-oxide based vulcanizing agents and a metal-oxide based vulcanizing agent for use as a watertight rubbery sealing material.

EP-A-0 179 937 discloses a water-swellable elastomer composition consisting essentially of a homogeneous mixture of an elastomer, a water-absorbent resin and a water-soluble resin. This composition exhibits a high degree of swelling and a high swelling rate when contacted with water. No mention is made in this document about a shape memory of the materials and of advantageous mechanical properties.

This object is achieved according to the claims. The dependent claims relate to preferred embodiments.

The filler content of these composite rubbers is preferably 10 to 120 mass parts per 100 mass parts of the rubber(s) used corresponding to 10 to 120 mass-%.

As rubbers, there are used natural rubber, butadiene-styrene rubbers, nitrile rubbers (acrylonitrile-butadiene rubbers), bromobutyl rubbers, ethylene-propylene rubbers, or chloroprene rubber.

Hydrogel fillers which are used are fillers consisting of particles of polymeric hydrogels based on physically or chemically crosslinked polymers or copolymers of monomethacrylates of glycols, polyols or dihydroxyethers, amides of methacrylic acid or their N-mono- and N,N-disubstituted derivatives, or multiblock copolymers of acrylonitrile with acrylamide or acrylic acid.

The preparation of these materials consists generally in the vulcanization of a mixture of the respective synthetic rubber with the powdery hydrogel by technical processes usual in the production of technical rubbers at temperatures of 15 to 150 °C.

The obtained water-swellable rubbers are composite materials consisting of two polymeric phases: A crosslinked rubber phase and a powdery hydrogel phase dispersed therein and being also slightly crosslinked. The structure of these new materials is thus very similar to common filled rubbers. Consequently, these materials exhibit, above all in the non-swollen state, properties characteristic for technical rubbers, namely a high elastic elongation and a considerable elongation at break. However, the different quality of powdery synthetic hydrogels, as compared with common fillers, gives them some completely new properties, the most important of them being their ability to swell in water and aqueous solutions. The degree of equilibrium swelling and the swelling rate are, at the same time, dependent both on the properties of the rubber matrix and on the content and composition of the hydrogel filler. This is why these properties can be controlled within a wide range, if desired.

Other important properties of the water swellable rubbers are their thermoformability and the shape memory of articles made therefrom. This makes it possible to change the shape of articles made from these water-swellable rubbers essentially by heating, forming in the heated state and cooling the new formed article. This newly prepared form is entirely stable at normal and slightly elevated temperatures. However, it can be perfectly changed to the original shape by a simple procedure, either by mere heating of the shaped article to a temperature above the softening temperature of the hydrogel filler (which is, as a rule, slightly above 100 °C), or by swelling with water. In the first case, the original shape and dimension of the article shaped on heating are recovered; in the other case, the original shape is obtained with appropriately magnified dimensions, which are mostly very slightly magnified.

In contrast to the known silicone rubber-hydrogel composite materials mentioned above, the water-swellable rubbers according to the invention exhibit better mechanical properties in the water-swollen state at the same hydrogel content. Thus, for example, the water-swelling rubber based on butadiene-styrene rubber, which is described in example 2, has in the non-swollen state approximately the same modulus at an elongation of 100 % as the silicon rubber-poly(HEMA) composite material, but a doubled tensile strength, and a more than three times higher elongation. The water swellable butadiene-styrene rubber contains in the swelling equilibrium the same amount of water as the silicone rubber-poly(HEMA) composite material swollen to equilibrium, but it has more than double the modulus at an elongation of 100 %, a more than three-times higher tensile strength and more than twice the elongation at break.

Still much better mechanical properties are obtained with swellable rubbers based on bromobutyl rubber and ethylene-propylene rubber, which have even substantially higher tensile strength at a water content of 7 % than without swelling with water. Like the silicone rubber-hydrogel composite materials, the water-swellable rubbers with a high water content, have mechanical properties inferior to those of the corresponding non-swollen materials, but considerably better than those of hydrogels based on methacrylic esters having the same water content.

The method for preparation of the water swellable rubbers is the same as for rubbers of common type. The technical equipment and procedures which are common in the preparation of rubber blends, and the usual vulcanization may be used for this purpose. A common procedure consists in thoroughly mixing the corresponding rubber with the hydrophilic powdery filler, addition of a suitable vulcanization agent, and thermoforming with vulcanization carried out at the same time.

The powdery hydrogel used may be prepared by methods suitable for the preparation of synthetic hydrogels in powdery form, for example, by precipitation copolymerization of 2-hydroxyethyl methacrylate (HEMA) with ethylene glycol dimethacrylate in toluene. Furthermore, it is possible to use powdery hydrogels obtained from the respective polymers in a bulk form by grinding or other dispersing methods, provided that size and structure of the particles obtained in this way are suitable with respect to the mechanical and swelling properties of the corresponding vulcanizate. The water swellable rubbers can be also prepared from rubber compounds containing, besides the powdery hydrogels, also other suitable fillers or further usual additives.

The composite rubbers of the present invention may be advantageously used, in the form of appropriately formed shaped articles, inter alia for many medical and pharmaceutical purposes, such as for medical implants, pharmaceutical articles comprising drugs, preferably water-soluble drugs, articles for controlled drug release, for intradermal drug applications, etc.

In the following, the invention will be further explained with reference to examples. Unless otherwise defined, all given percentages of amounts and ratios are by mass.

### Example 1

2-Hydroxyethyl methacrylate (HEMA) containing 3 % of ethylene glycol dimethacrylate was dissolved in toluene to a 15 % solution. Then 0.3 mol-% of 2,2'-azobis(isobutyronitrile), based on HEMA, was added, and the mixture was heated to 60 °C and stirred for 2 h. The resulting pasty product was filtered under vacuum, washed with toluene and dried. The powdery poly(HEMA) prepared in this way was blended with natural rubber and other components according to the following formulation in a two-roll mill:

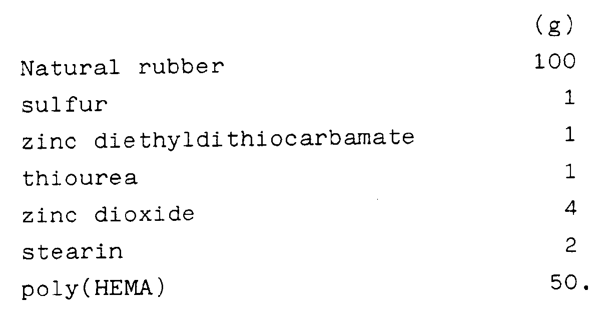

The blend prepared in this way was pressed to sheets of about 1 mm thickness at 110 °C in a hand press. Vulcanization time was 35 min. The obtained sheets from water-swellable rubber based on natural rubber had, in the nonswollen (dry) state, a modulus at 100 % elongation equal to 1.24 MPa, a tensile strength of 6.96 MPa and an elongation at break of 470 %. After immersion in water for 25 days, the mass was increased by 14.1 % due to swelling, whereas the modulus at 100 % elongation decreased to 0.52 MPa, and tensile strength and elongation at break were increased to 8.7 MPa and 730 %, respectively.

### Example 2

A rubber stock was prepared by the procedure described in example 1, with the exception that a nitrile rubber was used instead of natural rubber. Sheets of approx. 1 mm thickness were pressed from this stock at 110 °C in a hand press. The vulcanization time was 15 min. The resulting sheets from water-swellable rubber based on nitrile rubber had, in the nonswollen (dry) state, a modulus at 100 % elongation of 1.54 MPa, a tensile strength of 1.84 MPa, and an elongation at break of 280 %. The mass was increased by swelling after 25 days of immersion in water by 17 %, whereas the modulus at 100 % elongation decreased to 0.66 MPa, and tensile strength and elongation at break were increased to 1.85 MPa and 500 %, respectively.

### Example 3

A rubber stock was prepared by the procedure described in example 1, with the exception that a butadiene-styrene rubber was used instead of natural rubber. Sheets of about 1 mm thickness were pressed from this stock at 110 °C in a hand press. The vulcanization time was 90 min. The resulting sheets from water-swellable rubber based on butadiene-styrene rubber had, in the nonswollen (dry) state, a modulus at 100 % elongation of 1.68 MPa, a tensile strength of 2.50 MPa, and an elongation at break of 780 %. Their mass was increased by swelling by 21 % after immersion in water for 25 days, whereas the modulus at 100 % elongation, the tensile strength, and the elongation at break decreased to 0.55 MPa, 1.75 MPa, and 740 %, respectively.

### Example 4

Powdery poly(HEMA) was prepared by the procedure described in example 1. It was then blended with bromobutyl rubber (BIIR) and dicumyl peroxide to a rubber stock according to the following formulation:

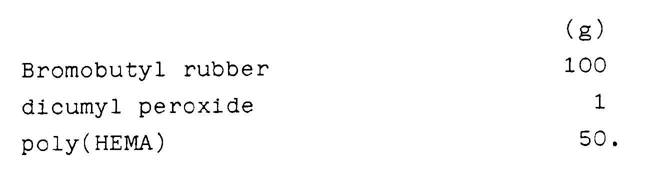

The stock was pressed to sheets of approx. 1 mm thickness at 145 °C in a hand press. The vulcanization time was 60 min. The resulting sheets from water-swellable rubber based on bromobutyl rubber had in the nonswollen (dry) state a modulus at 100 % elongation of 1.68 MPa, a tensile strength of 3.42 MPa, and an elongation at break of 510 %. The mass was increased by 7.87 % by swelling in water for 25 days, whereas the modulus at 100 % elongation decreased to 1.34 MPa, and tensile strength and elongation at break were increased to 5.03 MPa and 560 %, respectively.

### Example 5

A rubber stock was prepared by the procedure described in example 4 with the exception that an ethylene-propylene rubber was used instead of bromobutyl rubber. Sheets of about 1 mm thickness were pressed from this stock at 145 °C in a hand press. The vulcanization time was 60 min. The resulting sheets from water-swellable rubber based on ethylene-propylene rubber had, in the nonswollen (dry) state, a modulus at 100 % elongation of 1.74 MPa, a tensile strength of 3.45 MPa, and an elongation at break of 960 %. After immersion in water for 25 days, the mass was increased by swelling by 6.32 %, whereas the modulus at 100 % elongation decreased to 1.41 MPa, and tensile strength and elongation at break increased to 4.58 MPa and 1040 %, respectively.

### Example 6

According to the procedure described in example 3 sheets of 1 mm thickness were prepared from water-swellable rubber based on butadiene-styrene rubber. Test pieces for the measurement of mechanical properties were prepared from these sheets according to Czechoslovak State Standard CSN 640605. The pieces were heated to 135 °C, stretched to 200 % of the original length, and cooled in the stretched state down to normal temperature. After the deformation force causing the elongation had been removed, only a partial relaxation of the deformed pieces occured, and the pieces remained permanently elongated to 172 ± 4 % of their original length. The permanent deformation remained completely unchanged for 7 days. By heating the deformed pieces to 135 °C, they acquired their original shape and size within 10 min. Another group of deformed test pieces was immersed into water where they resumed their original shape within 15 h, whereas their linear dimensions were by 7 ± 0.6 % larger than the original ones due to swelling.

### Example 7

A monomer mixture consisting of 2-hydroxyethyl methacrylate (HEMA), methacrylic acid (MAA), and ethylene glycol dimethacrylate in a ratio of 75:23:2 was dissolved in toluene to a 15 % solution; then, 0.3 % of 2,2'-azo-bis(isobutyronitrile) was added, based on the total amount of monomers, and the mixture was heated for 3 h to 60 °C. The resulting pasty product was filtered off, washed with toluene, and dried. The powdery, slightly crosslinked HEMA-MAA copolymer prepared in this way was mixed in a two-roll mill with butadiene-styrene rubber and other components according to the following formulation:

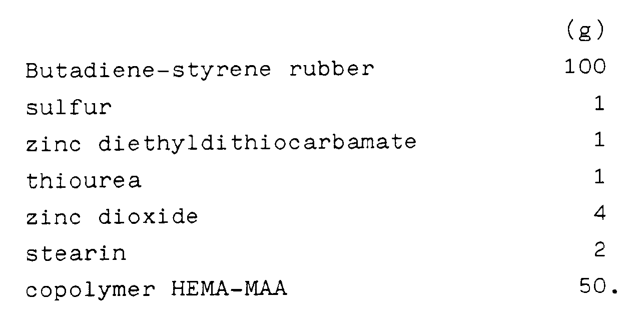

From this blend, sheets of about 1 mm thickness were pressed in a hand press at 110 °C. The vulcanization time was 90 min. The sheets of water-swellable rubber had in the nonswollen (dry) state a modulus at 100 % elongation 1.48 MPa, a tensile strength of 2.30 MPa, and an elongation at break of 840 %. After immersion in phosphate buffer of pH 7.4 for 10 days, the mass was increased by 210 % due to swelling, whereas the modulus at 100 % elongation, the tensile strength, and the elongation at break were decreased to 0.18 MPa, 0.35 MPa, and 620 %, respectively.

### Example 8

A rubber stock was prepared by the procedure described in example 7 with the exception that natural rubber was used instead of butadiene-styrene rubber. Sheets of about 1 mm thickness were pressed from this stock in a hand press at 110 °C. The vulcanization time was 35 min. The prepared sheets from water-swellable rubber had in the nonswollen (dry) state a modulus at 100 % elongation equal to 0.61 MPa, a tensile strength of 3.39 MPa, and an elongation at break of 350 %. The mass of the sheets was increased by 180 % due to swelling after immersion in a phosphate buffer of pH 7.4 for 7 days, whereas the modulus at 100 % elongation, the tensile strength, and the elongation at break were decreased to 0.18 MPa, 0.53 MPa, and 300 %, respectively.

### Example 9

A slightly crosslinked powdery HEMA-MAA copolymer was synthesized according to the procedure described in example 7. This copolymer was used for the preparation of a rubber stock having the following composition:

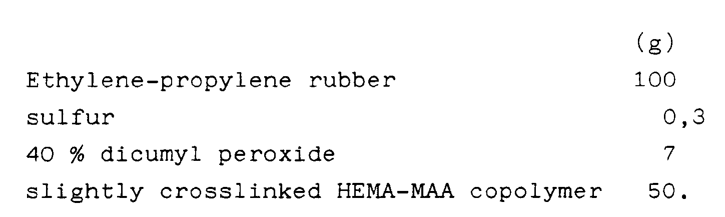

Sheets of about 1 mm thickness were pressed from the blend prepared in this way in a hand press at 145 °C. The vulcanization time was 60 min. The resulting sheets of water-swellable rubber based on ethylene-propylene rubber had in the nonswollen (dry) state a modulus at 100 % elongation equal to 3.92 MPa, a tensile strength of 7.32 MPa, and an elongation at break of 380 %. The mass was increased by 53 % due to swelling after immersion in water for 7 days, whereas the modulus at 100 % elongation, the tensile strength, and the elongation at break were decreased to 0.57 MPa, 2.17 MPa, and 350 %, respectively.

## Claims

1. Composite rubbers swellable with water and aqueous solutions, consisting of a vulcanized rubber matrix on the basis of one or more rubbers vulcanizable at temperatures up to 150 °C and selected from natural rubber, butadiene-styrene rubbers, acrylonitrile-butadiene rubbers, bromobutyl rubbers, and ethylene-propylene rubbers, and one or more powdery hydrogel fillers consisting of one or more polymeric hydrogels based on physically or chemically crosslinked polymers selected from polymers and copolymers of monomethacrylates of glycols, polyols and dihydroxyethers, amides of methacrylic acid and acrylic acid, and their N-mono- and N,N-disubstituted derivatives, and multiblock copolymers of acrylonitrile with acrylamide or acrylic acid dispersed therein, and optionally other, usual additives, silicone rubbers being excluded.

2. The composite rubbers according to claim 1, characterized in that the hydrogel filler content is 10 to 120 mass-%, based on the mass of the rubber(s) of the rubber matrix.

3. The composite rubbers according to claims 1 and 2, characterized in that the hydrogel filler consists of poly(HEMA) and/or a copolymer of HEMA with ethylene glycol dimethacrylate.

4. A method for preparing the composite rubbers according to claims 1 to 3, characterized by
(A) blending one or more powdery hydrogel fillers into one or more non-crosslinked rubbers vulcanizable at temperatures up to 150 °C,
(B) shaping of the unvulcanized blend,
and
(C) crosslinking/vulcanizing the blend at a temperature up to 150 °C, preferably at a temperature of 15 to 150 °C.

5. The method according to claim 4, characterized in that the hydrogel filler is used in an amount of 10 to 120 mass-%, based on the mass of the rubber(s) of the rubber matrix.

6. The method according to claim 4 or 5, characterized in that the rubbers of the rubber matrix are selected from natural rubber, butadiene-styrene rubbers, acrylonitrile-butadiene rubbers, bromobutyl rubbers, and ethylene-propylene rubbers, and/or the hydrogel fillers are selected from polymers and copolymers of monomethacrylates of glycols, polyols and dihydroxyethers, amides of methacrylic acid and acrylic acid, and their N-mono- and N,N-disubstituted derivatives, and a multiblock copolymers of acrylonitrile with acrylamide or acrylic acid.

7. Shaped articles comprising or consisting of one or more composite rubbers according to claims 1 to 3, the composite rubbers preferably comprising drugs, preferably water-soluble drugs.

8. Use of the shaped articles according to claim 7 as or for the production of medical implants, pharmaceutical articles for controlled drug release and articles for intradermal drug release.

## Patentansprüche

1. Mit Wasser und wäßrigen Lösungen quellfähige Verbundkaut schukarten aus einer aus vulkanisiertem Kautschuk bestehenden Grundmasse auf der Basis einer oder mehrerer Kautschukarten, die bei Temperaturen von bis zu 150 °C vulkanisierbar sind und ausgewählt sind aus Naturkautschuk, Butadien-Styrolkautschukarten, Acrylnitril-Butadienkautschukarten, Brombutylkautschukarten und Ethylen-Propylenkautschukarten, und auf der Basis eines oder mehrerer darin dispergierter pulverförmiger Hydrogelfüllstoffe aus einem oder mehreren polymeren Hydrogelen auf der Basis physikalisch oder chemisch vernetzter Polymere, ausgewählt aus Polymeren und Copolymeren von Monomethacrylaten von Glycolen, Polyolen und Dihydroxyethern, Amiden von Methacrylsäure und Acrylsäure und deren N-mono- und N,N-disubstituierten Derivaten sowie Multiblockcopolymeren von Acrylnitril mit Acrylamid oder Acrylsäure, und wahlweise aus anderen üblichen Zusätzen, außer Silikonkautschukarten.

2. Verbundkautschukarten nach Anspruch 1, dadurch gekennzeichnet, daß der Hydrogelfüllstoffgehalt 10 bis 120 Massen-% beträgt auf der Basis des Kautschuks (der Kautschukarten) der Kautschukgrundmasse.

3. Verbundkautschukarten nach Anspruch 1 und 2, dadurch gekennzeichnet, daß der Hydrogelfüllstoff aus Poly(HEMA) und/oder einem Copolymer von HEMA mit Ethylenglycoldimethacrylat besteht.

4. Verfahren zur Herstellung von Verbundkautschukarten nach Anspruch 1 bis 3, gekennzeichnet durch
(A) Einmischen eines oder mehrerer pulverförmiger Hydrogelfüllstoffe in eine oder mehrere nicht vernetzte Kautschukarten, die bei Temperaturen von bis zu 150 °C vulkanisierbar sind,
(B) Formen des unvulkanisierten Gemischs und
(C) Vernetzen/Vulkanisieren des Gemischs bei einer Temperatur von bis zu 150 °C.

5. Verfahren nach Anspruch 4, dadurch gekennzeichnet, daß der Hydrogelfüllstoff in einer Menge von 10 bis 120 Massen-% verwendet wird auf der Basis der Masse des Kautschuks (der Kautschukarten) der Kautschukgrundmasse.

6. Verfahren nach Anspruch 4 oder 5, dadurch gekennzeichnet, daß die Kautschukarten der Kautschukgrundmasse gewählt werden aus Naturkautschuk, Butadien-Styrolkautschukarten, Acrylnitril-Butadienkautschukarten, Brombutylkautschukarten und Ethylen-Propylenkautschukarten, und/oder daß die Hydrogelfüllstoffe ausgewählt werden aus Polymeren und Copolymeren von Monomethacrylaten von Glycolen, Polyolen und Dihydroxyethern, Amiden von Methacrylsäure und Acrlysäure und deren N-mono- und N,N-disubstituierten Derivaten sowie Multiblockcopolymeren von Acrylnitril mit Acrylamid oder Acrylsäure.

7. Geformte Gegenstände mit oder aus einer oder mehreren Verbundkautschukarten nach Anspruch 1 bis 3, wobei die Verbundkautschukarten vorzugsweise Arzneimittel umfassen, die vorzugsweise wasserlöslich sind.

8. Verwendung der geformten Gegenstände nach Anspruch 7 als medizinische Implantate, pharmazeutische Artikel für eine kontrollierte Arzneimittelfreisetzung und Artikel für eine intradermale Arzneimittelfreisetzung, oder für die Herstellung derselben.

## Revendications

1. Caoutchoucs composites pouvant gonfler à l'eau et aux solutions aqueuses, constitués d'une matrice de caoutchouc vulcanisé à base d'un ou plusieurs caoutchoucs vulcanisables à des températures allant jusqu'à 150°C, et choisis parmi l'ensemble comprenant le caoutchouc naturel, les caoutchoucs butadiène-styrène, les caoutchoucs acrylonitrile-butadiène, les caoutchouc de bromobutyle et les caoutchouc éthylène-propylène, et une ou plusieurs charges pulvérulentes du type hydrogel qui y sont dispersées, constituées d'un ou plusieurs hydrogels polymères à base de polymères à réticulation physique ou chimique choisis parmi l'ensemble comprenant les polymères et les copolymères des monométhacrylates de glycol, de polyols et de dihydroxyéthers, d'amides de l'acide méthacrylique et de l'acide acrylique, et de leurs dérivés N-mono- et N,N-disubstitués, et les copolymères multiséquencés de l'acrylonitrile et de l'acrylamide ou de l'acide acrylique, et éventuellement d'autres additifs usuels, à l'exclusion des caoutchoucs siliconés.

2. Caoutchoucs composites selon la revendication 1, caractérisés en ce que la charge d'hydrogel est présente en une quantité de 10 à 120 % en masse par rapport à la masse du ou des caoutchoucs de la matrice de caoutchouc.

3. Caoutchoucs composites selon les revendications 1 et 2, caractérisés en ce que la charge d'hydrogel est constituée d'un poly(HEMA) et/ou d'un copolymère de HEMA et de diméthacrylate d'éthylèneglycol.

4. Procédé pour préparer les caoutchoucs composites selon les revendications 1 à 3, caractérisé en ce qu'il consiste :
(A) à introduire par mélange une ou plusieurs charges d'hydrogel en poudre dans un ou plusieurs caoutchoucs non réticulés, vulcanisables à des températures allant jusqu'à 150°C,
(B) à façonner le mélange non vulcanisé, et
(C) à réticuler/vulcaniser le mélange à une température allant jusqu'à 150°C et de préférence à une température de 15 à 150°C.

5. Procédé selon la revendication 4, caractérisé en ce que la charge d'hydrogel est utilisée en une quantité de 10 à 120 % en masse par rapport à la masse du ou des caoutchoucs de la matrice de caoutchouc.

6. Procédé selon la revendication 4 ou 5, caractérisé en ce que les caoutchoucs de la matrice de caoutchouc sont choisis parmi l'ensemble comprenant le caoutchouc naturel, les caoutchoucs butadiène-styrène, les caoutchoucs acrylonitrile-butadiène, les caoutchoucs de bromobutyle et les caoutchoucs éthylène-propylène, et/ou les charges d'hydrogel sont choisies parmi l'ensemble comprenant les polymères et copolymères de monométhacrylates de glycol, de polyols et de dihydroxyéther, d'amides de l'acide méthacrylique et de l'acide acrylique, et de leurs dérivés N-mono- et N,N-disubstitués, et les copolymères multiséquencés de l'acrylonitrile et de l'acrylamide ou de l'acide acrylique.

7. Articles façonnés comprenant un ou plusieurs caoutchoucs composites selon les revendications 1 à 3 ou constitués de ces caoutchoucs, les caoutchoucs composites comprenant de préférence des médicaments, et plus particulièrement des médicaments hydrosolubles.

8. Utilisation des articles façonnés selon la revendication 7, en tant qu'implants médicaux, articles pharmaceutiques pour assurer une libération retardée, et articles pour une libération intradermique de médicaments,ou pour la production de ces implants et articles.
